## Europäisches Patentamt
## European Patent Office
## Office européen des brevets

(11) Veröffentlichungsnummer: **0 280 642**
**A1**

(12) # EUROPÄISCHE PATENTANMELDUNG

(21) Anmeldenummer: 88730007.7

(22) Anmeldetag: 13.01.88

(51) Int. Cl.⁴: **A 61 F 2/32**

(30) Priorität: 15.01.87 DE 3701198

(43) Veröffentlichungstag der Anmeldung:
31.08.88 Patentblatt 88/35

(84) Benannte Vertragsstaaten: **BE FR GB SE**

(71) Anmelder: **Buse, Harry, Prof. Dr. med.**
**Kaunstrasse 5**
**D-1000 Berlin 37 (DE)**

(72) Erfinder: **Buse, Harry, Prof. Dr. med.**
**Kaunstrasse 5**
**D-1000 Berlin 37 (DE)**

(74) Vertreter: **Maikowski, Michael, Dipl.-Ing. Dr.**
**Xantener Strasse 10**
**D-1000 Berlin 15 (DE)**

(54) **Hüftschaft-Implantat.**

(57) Die Erfindung betrifft ein Hüftschaft-Implantat 2 mit einem Kragen 5, der eine ballige, insbesondere als Kugelzone ausgebildete Unterseite 6 aufweist.

EP 0 280 642 A1

## Beschreibung

## Hüftschaft-Implantat

Die Erfindung betrifft ein Hüftschaft-Implantat nach dem Oberbegriff des Patentanspruchs 1.

Es sind Hüftschaft-Implantate mit Kragen bekannt, dessen Funktion darin besteht, die auf den Kopf des Hüftschaft-Implantates einwirkende Körperlast auf den Knochenschaft des Oberschenkels zu übertragen. Ein derartiges Implantat wird unter anderem in der DE-OS 28 39 661 beschrieben.Da die auf den Kragen einwirkende Lastresultierende an der Kragenauflage des Knochens Scherkräfte erzeugt, erfolgt eine wechselseitige Verschiebung von Kragen- und Knochenfläche, wodurch eine Lockerung der Prothese im Knochen herbeigeführt werden kann. Dazu kommt, daß die Kragenauflage bei den bekannten varischen Schäften, die bei diesen vorhandene Momentenwirkung verstärkt.

Um diese Nachteile zu beseitigen, wurden kragenlose Implantate entwickelt, bei denen die Lasteintragung in den Oberschenkelknochen nicht über eine Kragenauflage sondern über die Umfangsspannung des Oberschenkelknochenköchers erfolgt. Diese Art der Lasteintragung in den Knochen ist jedoch nicht sinnvoll, sondern zeigt schädliche Wirkungen auf den Knochen. Der Knochenmantel des Oberschenkels ist den hohen Umfangsspannungen aus der Körperlast weitgehend nicht gewachsen. Wie die Erfahrung zeigt, kommt es vielfach schon während der Operation beim Einkeilen des Prothesenschaftes in den Knochen zu Umfangsspannungsbrüchen des Knochenmantels. Solche Umfangsspannungsbrüche ereignen sich auch nach der Operation unter der täglichen Belastung, vor allem bei übergewichtigen Patienten, bei Patienten mit verminderter Knochenfestigkeit infolge Krankheiten, die mit einer Entkalkung des Knochens einhergehen (Osteoporose), bei alten Menschen ganz allgemein, welche infolge ihres Alters eine verminderte Knochenfestigkeit aufweisen (altersbedingte Osteoporose) und sogar bei jüngeren Patienten, die zwar keine verminderte Knochenfestigkeit aufweisen, aber zu großer körperlicher Aktivität neigen (z.B. Sportler).

Dementsprechend hat man beobachtet, daß kragenlose Prothesenschäfte auch noch nach der Operation zunehmend tiefer in den Knochen einsinken, was monatelang anhaltende Schmerzen verursachen kann. Daher ist es allgemein üblich, den Patienten eine Vollbelastung ihrer Prothesen in den ersten Monaten nach der Operation zu untersagen.

Auch zeigt die bei kragenlosen Prothesenschäften bewirkte Eintragung der Körperlast ausschließlich über den Knochenköcher eine auch biologisch nachteilige Wirkung. Es is bekannt, daß der Knochenmantel, der stärkere Umfangsspannungen nicht verträgt, umgekehrt hohe Drucklasten (senkrecht zum Knochenquerschnitt) nicht nur toleriert, sondern sogar verlangt (biologische Wirkung axialer Druckbeanspruchung, sogenanntes Wolfsches Transformationsgesetz der Knochenformation). Speziell der Oberschenkelhals und der Oberschenkelknochen sind, wie sich aus seiner trajektoriellen Struktur ablesen läßt, biologisch darauf angelegt, Drucklasten zu übernehmen. Folglich führt die Abwesenheit von axialer Druckbeanspruchung auf den Knochenmantel, wie sie bei kragenlosen Schäften vorliegt, dazu, daß der Knochen atrophiert, d.h. er wird entmineralisiert und bildet sich unter Umständen sogar vollständig zurück, wie man dies bei kragenlosen Prothesen an der Resektionsfläche des Schenkelhalses gesetzmäßig beobachten kann.

Der Erfindung liegt die Aufgabe zugrunde, ein Hüftschaft-Implantat mit einem Kragen zu schaffen, bei dem eine wechselseitige Verschiebung von Kragen- und Knochenfläche verhindert wird.

Erfindungsgemäß wird diese Aufgabe durch die technische Lehre des Patentanspruchs 1 gelöst. Bei Verwendung eines Kragens mit einer balligen Unterseite wird die Kragenauflage des Knochens als komplementäre Auflagefläche mittels einer entsprechend geformten Fräse ausgebildet. Die ballige Unterseite des Kragens sitzt in der komplementären Auflagefläche im Oberschenkelknochen derart, daß eine translatorische Verschiebung zwischen diesen Knochen durch Scherkräfte durch das formschlüssige Auflager verhindert wird.

Mit besonderem Vorteil kann die Unterseite des Kragens als Kugelzone ausgebildet sein und dabei kann der Mittelpunkt der Kugelzone der Mittelpunkt des Kopfes des Hüftschaft-Implantates sein. Dies bringt den erheblichen Vorteil mit sich, daß die Unterseite des Kragens derart beschaffen ist, daß jeder mögliche Lasteinfall an der Kragenauflagefläche des Knochens senkrecht zu dieser erfolgt, so daß die Scherkräfte, die eine Verschiebung hervorrufen könnten, sehr klein, fast zu Null werden. Es treten fast ausschließlich erwünschte Druckkräfte auf.

Es ist auch möglich, die sich am Oberschenkel abstützende Unterseite des Kragens als Kegelmantelfläche auszubilden, wobei die Kragenauflage des Oberschenkelknochens als komplementäre Kegelmantelfläche ausgebildet wird.

Es kann manchmal nicht ausgeschlossen werden, daß aus irgendwelchen Gründen am Kopf des Hüftschaft-Implantates Kräfte auftreten, die eine relative Bewegung des Endes des Schaftes im Knochen bewirken. An der Knochenauflage kann daher eine Bewegung auftreten, wie sie zwischen einer Lagerschale und einem Lagerkopf möglich ist. Gemäß weiterer Erfindung kann mit Vorteil am Hüftschaft-Implantat das Merkmal, daß die sich am Oberschenkelknochen abstützende Unterseite des Kragens ballig, insbesondere als Kugelzone, deren Mittelpunkt im Mittelpunkt des Kopfes liegt, ausgebildet ist, mit dem Merkmal kombiniert werden, daß der Schaft einen zumindest teilweise elastisch und/oder plastisch deformierbaren Paßsitzabschnitt aufweist. Mit Vorteil kann dabei der Schaft ein Verbundschaft sein und der Paßsitzabschnitt ein lösbar mit dem Schaft verbundenes Paßsitzstück.

Das zumindest teilweise elastisch und/oder plastisch deformierbare Paßsitzstück erleichtert zunächst durch eine Selbstzentrierung das Einführen dieses Hüftschaft- Implantates in das Knocheninnere und gewährleistet einen formschlüssigen Paßsitz. Durch diesen formschlüssigen Paßsitz im implantierten Zustand werden alle Belastungen aufgenommen, die ein schädliches Moment, wie es im vorstehenden erläutert wurde, am Implantat erzeugen könnten. Die sichere Verhinderung einer relativen Bewegung des Schaftendes im Knochen wird mit der speziellen Ausbildung der sich am Oberschenkelknochen abstützenden Unterseite des Kragens erreicht. Die Relativbewegungen zwischen Implantat und Knochen und damit eine wechselseitige Bewegung zwischen Kragen und Knochenauflagefläche werden mit großer Sicherheit verhindert.

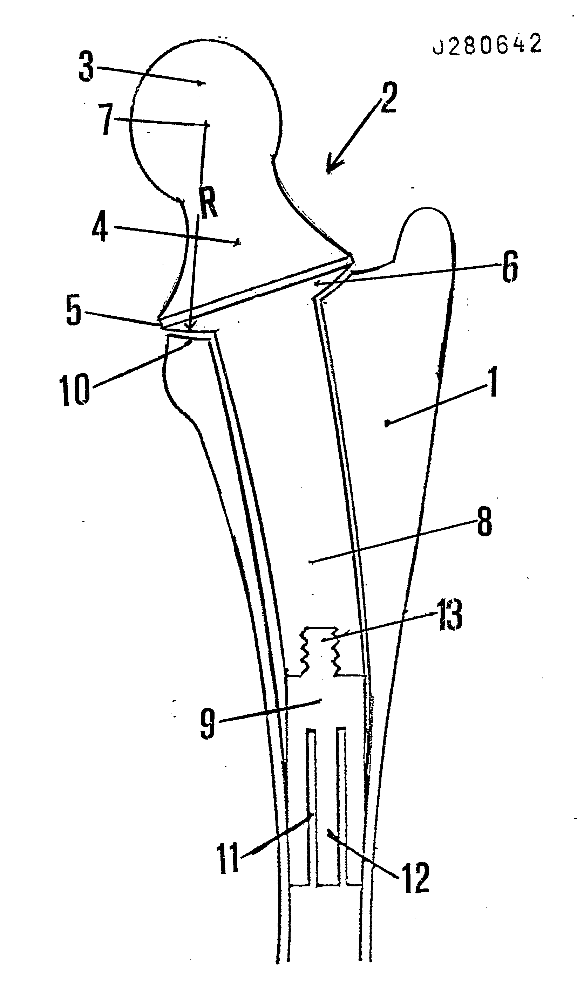

Ein Ausführungsbeispiel der Erfindung soll unter Bezugnahme auf die Zeichnung erläutert werden, die eine schematische Schnittansicht eines implantierten Hüftschaft-Implantates darstellt.

Das Hüftschaft-Implantat 2 weist einen Kopf 3 auf, der über einen Halsteil 4 in einen Kragen 5 übergeht. Dieser Kragen 5 hat die Aufgabe, in den Oberschenkelknochen 1 Druckkräfte einzuleiten. Die Unterseite 6 des Kragens ist beim dargestellten Ausführungsbeispiel als ballige Kugelzone ausgebildet. Der Mittelpunkt der Kugel, die diese Kugelzone aufweist, ist der Mittelpunkt 7 des Kugelkopfes 3.

Beim dargestellten Ausführungsbeispiel ist die Kragenauflagefläche 10 des Oberschenkelknochens 1 mittels einer Kugelfräse derart gefräst, daß diese Kragenauflagefläche 10 die Form einer Kugelzone hat.

Da die Lastresultierenden der vom Körper auf den Kugelkopf 3 aufgebrachten Kräfte durch den Mittelpunkt 7 der Kugel 3 hindurchgehen, stehen diese resultierenden R auf der Unterseite 6 des Kragens 5 und auf der Kragenauflagefläche 10 des Knochens 1 senkrecht, so daß keine Scherkräfte erzeugt werden. Eine relative Verschiebung zwischen der Unterseite 6 des Kragens 5 und der Kragenauflagefläche 10 des Oberschenkelknochen 1 wird auf diese Weise sicher verhindert. Da keine wechselseitigen Verschiebungen von Kragen- und Knochenfläche auftreten, erfolgt auch keine Lockerung der Prothese im Knochen.

Mit besonderem Vorteil kann der Schaft 8 des HüftschaftImplantats 2 einen zumindest teilweise elastisch und/oder plastisch deformierbaren Paßsitzabschnitt 9 aufweisen. Der Paßsitzabschnitt kann als Paßsitzstück 9 ausgebildet sein und einen runden Querschnitt aufweisen. Zur Erzielung einer elastischen Deformierbarkeit, zumindest eines Teiles des Paßsitzstückes 9 sind beim dargestellten Ausführungsbeispiel vom freien Ende aus sich erstreckende Schlitze 11 vorgesehen, so daß elastisch deformierbare Zungen 12 verbleiben. Bei 13 ist eine Schraubverbindung zwischen dem Paßsitzstück 9 und dem Schaft 8 dargestellt. Das Ende des Schaftes kann satt im Knochen sitzen und extreme Momentenbelastungen aufnehmen.

## Patentansprüche

1. Hüftschaft-Implantat, insbesondere für zementloses Implantieren mit einem Kopf mit Halsteil und Kragen und einem Schaft, dadurch gekennzeichnet, daß die sich am Oberschenkelknochen (1) abstützende Unterseite (6) des Kragens (5) ballig ausgebildet ist.

2. Hüftschaft-Implantat nach Anspruch 1, dadurch gekennzeichnet, daß die Unterseite (6) des Kragens (5) als Kugelzone ausgebildet ist.

3. Hüftschaft-Implantat nach Anspruch 2, dadurch gekennzeichnet, daß der Mittelpunkt (7) der Kugelzone in den Mittelpunkt des Kopfes (3) fällt.

4. Hüftschaft-Implantat, insbesondere für zementloses Implantieren mit einen Kopf mit Halsteil und Kragen und einem Schaft, dadurch gekennzeichnet, daß die sich am Oberschenkelknochen (1) abstützende Unterseite (6) des Kragens (5) als Kegelmantelfläche ausgebildet ist.

5. Hüftschaft-Implantat, insbesondere für zementloses Implantieren mit einem Kopf mit Halsteil und Kragen und einem Schaft, gekennzeichnet durch die Kombination der folgenden Merkmale:

    a) die sich am Oberschenkelknochen (1) abstützende Unterselte (6) des Kragens (5) ist ballig, insbesondere als Kugelzone, deren Mittelpunkt (7) mit Mittelpunkt des Kopfes (3) zusammenfällt, ausgebildet

    b) und der Schaft (8) einen zumindest teilweise elastisch und/oder plastisch deformierbaren Paßsitzabschnitt (9) aufweist.

6. Hüftschaft-Implantat nach Anspruch 5, dadurch gekennzeichnet, daß der Schaft (8, 9) ein Verbundschaft ist und daß der Paßsitzabschnitt ein lösbar mit dem Schaft (8) verbundenes Paßsitzstück ist

7. Hüftschaft-Implantat nach Anspruch 6, dadurch gekennzeichnet, daß das Paßsitzstück (9) aus Metall oder Kunststoff besteht, einen runden rohrförmigen Querschnitt hat und vom freien Ende ausgehende Längsschlitze aufweist.

0280642

**Europäisches Patentamt**

**EUROPÄISCHER RECHERCHENBERICHT**

Nummer der Anmeldung

EP 88730007.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - B2 - 2 555 717 (EICHLER)<br>* Anspruch 1; Fig. 1 *<br>-- | 1 | A 61 F 2/32 |
| A | US - A - 4 266 302 (TORNIER)<br>* Anspruch 1; Fig. 1 *<br>-- | 1 | |
| P,A | EP - A2 - 0 222 979 (GMT)<br>* Zusammenfassung; Fig. 1 *<br>---- | 1 | |

**RECHERCHIERTE SACHGEBIETE (Int. Cl.4)**

A 61 F

Der vorliegende Recherchenbericht wurde für alle Patentansprüche erstellt.

| Recherchenort | Abschlußdatum der Recherche | Prüfer |
|---|---|---|
| WIEN | 08-06-1988 | MIHATSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82